# EUROPÄISCHE PATENTANMELDUNG

(11) **EP 0 752 417 A1**
(43) Veröffentlichungstag der Anmeldung: **08.01.1997**
(21) Anmeldenummer: 96110359.5
(22) Anmeldetag: 27.06.1996
(51) Int. Cl.: C07C 275/20

(54) **Verfahren zur Herstellung von N-Alkenylharnstoffen**

(30) Priorität: 06.07.1995 DE 19524619
(71) Anmelder: BASF AKTIENGESELLSCHAFT, 67056 Ludwigshafen (DE)
(72) Erfinder: Henkelmann, Jochem, Dr., 68165 Mannheim (DE); Heider, Marc, Dr., 67433 Neustadt (DE); Rühl, Thomas, Dr., 67227 Frankenthal (DE)

(57) **Zusammenfassung**

Verfahren zur Herstellung von N-Alkenylharnstoffen der allgemeinen Formel I 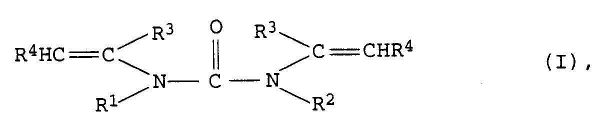 in der
- R¹,R²: Wasserstoff, C₁- bis C₄₀-Alkyl, C₂- bis C₄₀-Alkenyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl, durch C₁- bis C₈-Alkyl, C₁- bis C₈-Alkoxy, Halogen ein- bis fünffach substituiertes Aryl, C₇- bis C₂₀-Alkylaryl, C₇- bis C₂₀-Aralkyl oder gemeinsam eine gegebenenfalls durch C₁- bis C₈-Alkyl ein- bis sechsfach substituierte C₂- bis C₁₀-Alkylendikette und
- R³,R⁴: Wasserstoff oder C₁- bis C₈-Alkyl
bedeuten, durch Umsetzung von Harnstoffen der allgemeinen Formel II 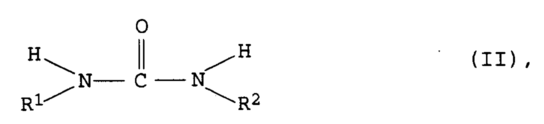 in der R¹ und R² die oben genannten Bedeutungen haben, mit einem Carbonsäurealkenylester der allgemeinen Formel III 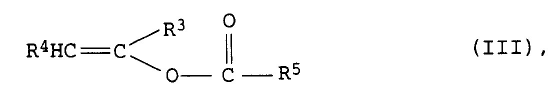 in der R³ und R⁴ die oben genannten Bedeutungen haben und R⁵ Wasserstoff, C₁- bis C₄₀-Alkyl, C₃- bis C₂₀-Cycloalkyl, C₄- bis C₂₀-Alkyl-cycloalkyl, Aryl, C₇- bis C₂₀-Alkylaryl, C₇- bis C₂₀-Aralkyl, durch C₁- bis C₈-Alkyl ein- bis dreifach substituiertes Aryl oder C₇- bis C₂₀-Aralkyl bedeutet, bei Temperaturen von 0 bis 180°C und Drücken von 0,01 bis 10 bar, indem man die Umsetzung in Gegenwart einer Base durchführt.

## Beschreibung

Die vorliegende Erfindung betrifft ein neues Verfahren zur Herstellung von N-Alkenylharnstoffen durch Umsetzung von Harnstoffen mit einem Carbonsäurealkenylester bei erhöhten Temperaturen in Gegenwart einer Base.

Die Herstellung von Divinylharnstoffen durch Umsetzung der Harnstoffe mit Acetylen ist in US-A-2 542 152 und DE-A-911 017 beschrieben.

Der Umgang mit Acetylen erfordert einen großen technischen Aufwand im Hinblick auf die Acetylenverfügbarkeit und den sicheren Umgang mit Acetylen. Besonders ist der sicheren Reaktionsführung Aufmerksamkeit zu schenken, da der für die Umsetzung von Imidazolen mit Acetylen verwendete Katalysator-Kaliumhydroxid einen Acetylenzerfall im Reaktionssystem auslösen kann. Dies haben jüngste Untersuchungen von Schildberg et al. gezeigt (Chem. Ing. Tech. 66 (1994) 1389 bis 1392).

Aus J. Macromol. Sci., Chem. (1971), 5, 1903 bis 1914 ist eine mehrstufige acetylenfreie Synthese durch Überführung der Harnstoffe in die Di-Natriumsalze, Umsetzung zu den Bis(β-dimethylaminoethyl)-Derivaten und Spaltung zu den Divinylderivaten durch Cope- oder Hofmann-Abbau bekannt. Dieses Verfahren ist umständlich und technisch ungeeignet.

Der vorliegenden Erfindung lag daher die Aufgabe zugrunde, den zuvor genannten Nachteilen abzuhelfen.

Demgemäß wurde ein neues und verbessertes Verfahren zur Herstellung von N-Alkenylharnstoffen der allgemeinen Formel I 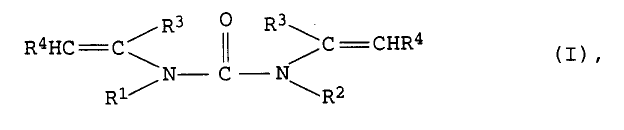 in der
- R¹,R²: Wasserstoff, C₁- bis C₄₀-Alkyl, C₂- bis C₄₀-Alkenyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl, durch C₁- bis C₈-Alkyl, C₁- bis C₈-Alkoxy, Halogen ein- bis fünffach substituiertes Aryl, C₇- bis C₂₀-Alkylaryl, C₇- bis C₂₀-Aralkyl oder gemeinsam eine gegebenenfalls durch C₁- bis C₈-Alkyl ein- bis sechsfach substituierte C₂- bis C₁₀-Alkylenkette und
- R³,R⁴: Wasserstoff oder C₁- bis C₈-Alkyl
bedeuten, durch Umsetzung von Harnstoffen der allgemeinen Formel II 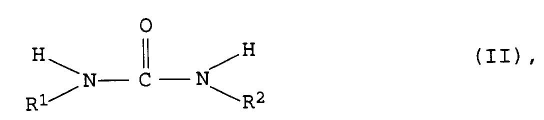 in der R¹ und R² die oben genannten Bedeutungen haben, mit einem Carbonsäurealkenylester der allgemeinen Formel III 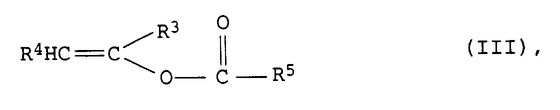 in der R³ und R⁴ die oben genannten Bedeutungen haben und R⁵ Wasserstoff, C₁- bis C₄₀-Alkyl, C₃- bis C₂₀-Cycloalkyl, C₄- bis C₂₀-Alkyl-cycloalkyl, Aryl, C₇- bis C₂₀-Alkylaryl, C₇- bis C₂₀-Aralkyl, durch C₁- bis C₈-Alkyl ein- bis dreifach substituiertes Aryl oder C₇- bis C₂₀-Aralkyl bedeutet, bei Temperaturen von 0 bis 180°C und Drücken von 0,01 bis 10 bar gefunden, welches dadurch gekennzeichnet ist, daß man die Umsetzung in Gegenwart einer Base durchführt.

Das erfindungsgemäße Verfahren läßt sich wie folgt durchführen:

Die Harnstoffe II und die Carbonsäurealkenylester III können kontinuierlich oder diskontinuierlich in Anwesenheit oder bevorzugt in Abwesenheit des Zusatzes eines inerten Lösungsmittels bei Temperaturen von 0 bis 180°C, bevorzugt 40 bis 150°C, besonders bevorzugt 50 bis 120°C und Drücken von 0,01 bis 10 bar, bevorzugt 0,1 bis 2 bar, besonders bevorzugt Atmosphärendruck (Normaldruck) in Gegenwart einer Base umgesetzt werden, wobei die Edukte in beliebiger Reihenfolge zusammengegeben werden können. So können die Ausgangsverbindungen II und III und die Base z.B. in einen Rührkessel gegeben und darin umgesetzt werden. Es ist auch möglich, die Ausgangsverbindungen II und III und die Base in einem Rohrreaktor z.B. in Riesel- oder Sumpffahrweise umzusetzen. Es hat sich als vorteilhaft erwiesen, die Reaktion in einem Strahldüsenreaktor vorzunehmen.

Als Basen eignen sich anorganische oder organische Basen, vorzugsweise Brönsted-Basen, z.B. Carbonate und Hydrogencarbonate der Alkali- und Erdalkalimetalle wie Natriumcarbonat, Kaliumcarbonat und Natriumhydrogencarbonat, quartäre Ammoniumcarbonate wie Tetramethylammoniumcarbonat, Amide wie Alkalimetallamide, beispielsweise Natriumamid und Kaliumamid, Hydroxide wie Alkalimetallhydroxide, beispielsweise Lithiumhydroxid, Natriumhydroxid und Kaliumhydroxid, Alkalimetallcarboxylate wie Natriumacetat, Alkoholate wie Alkalimetallalkoholate, beispielsweise Natriummethanolat, Natriumethanolat, Kaliummethanolat und Kalium-tert.-butanolat. Kaliumhydroxid kann auch zusammen mit Kronenethern wie 18-Krone-6 verwendet werden.

Als Basen eignen sich weiterhin Ammoniak, primäre, sekundäre und tertiäre Amine, bevorzugt tertiäre Amine. Die Amine können aliphatische oder aromatische Reste tragen, beispielsweise Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo-[5.4.0]-undec-7-en, aliphatische und aromatische Reste tragende Amine wie 1,8-Bis(dimethylamino)-naphthalin und 4-N,N-Dimethylaminopyridin und heterocyclische Amine wie N-Alkylimidazole und N-Arylimidazole. Weiterhin kommen Amide wie Dialkylcarbonsäureamide, z. B. Dibutylformamid, in Betracht. Das erfindungsgemäße Verfahren läßt sich auch in Gegenwart von basischen Ionenaustauschern, die in der Regel aus sulfonierten Styrol-Divinylbenzol-Copolymerisaten bestehen wie Amberlite®, Lewatit® und Puralit®, und in Gegenwart von basischen Zeolithen wie Hydrotalcit durchführen.

Das Molverhältnis von Carbonsäurealkenylester III zum Harnstoff II beträgt in der Regel 0,1:1 bis 10:1, bevorzugt 0,9:1 bis 5:1, besonders bevorzugt 2:1 bis 2,2:1.

Das Molverhältnis von Base zum Harnstoff II beträgt in der Regel 0,1:1 bis 10:1, bevorzugt 0,2:1 bis 4:1, besonders bevorzugt 0,2:1 bis 2:1.

Als inerte Lösungsmittel eignen sich beispielsweise aprotische Lösungsmittel z.B. Ether wie Tetrahydrofuran, aromatische Kohlenwasserstoffe wie Toluol und Xylol, Ketone wie Aceton, weiterhin Acetonitril, Hexamethylphosphorsäuretriamid, Sulfolan, Dimethylsulfoxid, Harnstoffe wie N,N'-Dimethylethylen-, N,N'-Dimethylpropylenharnstoff und Tetrabutylharnstoff. Die Menge liegt in der Regel bei 5 bis 300 Gew.-%, bevorzugt 10 bis 100 Gew.-%, besonders bevorzugt 10 bis 30 Gew.-% bezogen auf den Gesamtansatz.

In der Regel ist die Reaktion nach 5 Minuten bis 8 Stunden beendet.

Das so erhaltene Reaktionsgemisch kann in an sich bekannter Weise aufgearbeitet werden. In der Regel wird der N-Alkenylharnstoff I destillativ abgetrennt. Der Destillationssumpf kann zur Freisetzung der organischen Basen aus den bei der Reaktion anfallenden Salzen mit Laugen wie Natronlauge versetzt werden. Wird in der erfindungsgemäßen Umsetzung ein 4-N,N Dialkylaminopyridin eingesetzt, so können die gebildeten Säuren und das Amin auch ohne Behandlung mit Basen voneinander getrennt werden. Die beigesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Die jeweils abgetrennten Basen können in die Reaktion zurückgeführt werden.

Die Substituenten R¹, R², R³, R⁴ und R⁵ in den Verbindungen I, II und III haben folgende Bedeutungen:
R¹,R²,R³,R⁴,R⁵
- Wasserstoff,
R¹,R²,R⁵
- C₃- bis C₂₀-Cycloalkyl, bevorzugt C₃- bis C₁₂-Cycloalkyl, besonders bevorzugt C₅- bis C₈-Cycloalkyl wie Cyclopentyl, Cyclohexyl, Cycloheptyl und Cyclooctyl,
- C₄- bis C₂₀-Alkyl-cycloalkyl, bevorzugt C₄- bis C₁₂-Alkyl-cycloalkyl, besonders bevorzugt C₅- bis C₁₀-Alkyl-cycloalkyl,
- Aryl wie Phenyl, 1-Naphthyl und 2-Naphthyl, bevorzugt Phenyl,
- C₇- bis C₂₀-Alkylaryl, bevorzugt C₇- bis C₁₆-Alkylaryl, bevorzugt C₇- bis C₁₂-Alkylphenyl wie 2-Methylphenyl, 3-Methylphenyl, 4-Methylphenyl, 2-Ethylphenyl, 3-Ethylphenyl und 4-Ethylphenyl,
- C₇- bis C₂₀-Aralkyl, bevorzugt C₇- bis C₁₆-Aralkyl, bevorzugt C₇- bis C₁₂-Phenalkyl wie Phenylmethyl, 1-Phenylethyl und 2-Phenylethyl,
R¹,R²
- C₁- bis C₄₀-Alkyl, bevorzugt C₁- bis C₁₂-Alkyl, besonders bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, n-Hexyl, iso-Hexyl, n-Heptyl, iso-Heptyl, n-Octyl und iso-Octyl,
- C₂- bis C₄₀-Alkenyl, bevorzugt C₂- bis C₁₂-Alkenyl, besonders bevorzugt C₂- bis C₈-Alkenyl wie Vinyl und Propenyl,
- C₄- bis C₂₀-Cycloalkyl-alkyl, bevorzugt C₄- bis C₁₂-Cycloalkyl-alkyl, besonders bevorzugt C₅- bis C₁₀-Cycloalkyl-alkyl,
- durch C₁- bis C₈-Alkyl, C₁- bis C₈-Alkoxy, Halogen ein- bis fünffach substituiertes Aryl, bevorzugt durch C₁- bis C₈-Alkyl, C₁- bis C₈-Alkoxy, Halogen ein- bis dreifach substituiertes Phenyl,
- durch C₁- bis C₈-Alkyl, C₁- bis C₈-Alkoxy, Halogen ein- bis fünffach substituiertes C₇- bis C₂₀-Alkylaryl, bevorzugt durch C₁- bis C₈-Alkyl, C₁- bis C₈-Alkoxy, Halogen ein- bis dreifach substituiertes C₇- bis C₁₆-Alkylphenyl,
- durch C₁- bis C₈-Alkyl, C₁- bis C₈-Alkoxy, Halogen ein- bis fünffach substituiertes C₇- bis C₂₀-Aralkyl, bevorzugt durch C₁- bis C₈-Alkyl, C₁- bis C₈-Alkoxy, Halogen ein- bis dreifach substituiertes C₇- bis C₁₆-Phenalkyl,
- gemeinsam eine C₂- bis C₁₀-Alkylendikette, bevorzugt eine C₂- bis C₈-Alkylendikette, besonders bevorzugt -(CH₂)₂-, -(CH₂)₃-, -(CH₂)₄- und -(CH₂)₅-, insbesondere -(CH₂)₂- und -(CH₂)₃-,
- gemeinsam eine gegebenenfalls durch C₁- bis C₈-Alkyl ein- bis sechsfach substituiertes C₂- bis C₁₀-Alkylendikette, bevorzugt gemeinsam eine gegebenenfalls durch C₁- bis C₄-Alkyl ein- bis dreifach substituiertes C₂- bis C₅-Alkylendikette,
R³,R⁴
- C₁- bis C₈-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
R⁵
- C₁- bis C₄₀-Alkyl, bevorzugt C₁- bis C₂₀-Alkyl, besonders bevorzugt Methyl, Ethyl, n-Propyl, iso-Propyl, n-Butyl, tert.-Butyl, n-Pentyl, n-Hexyl, n-Heptyl, n-Octyl, n-Decyl, n-Dodecyl und n-Octadecyl,
- durch C₁- bis C₈-Alkyl ein- bis dreifach substituiertes Aryl, bevorzugt durch C₁- bis C₄-Alkyl ein- bis dreifach substituiertes Phenyl wie 2-Methylphenyl,
- durch C₁- bis C₈-Alkyl ein- bis dreifach substituiertes C₇- bis C₂₀-Aralkyl, bevorzugt durch C₁- bis C₄-Alkyl ein- bis dreifach substituiertes C₇- bis C₁₆-Phenalkyl.

Als Carbonsäurealkenylester III seien bevorzugt genannt: Vinylformiat, Vinylacetat, Vinylpropionat, Vinylstearat (Stearinsäurevinylester oder Octadecansäurevinylester), Vinylpivalat (Pivalinsäurevinylester oder 2,2-Dimethylpropionsäurevinylester) und 4-tert.-Butylbenzoylvinylester.

Die Carbonsäurealkenylester III sind käuflich oder nach bekannten Methoden herstellbar, beispielsweise durch Addition von Carbonsäuren an Acetylen oder durch Acetoxilierung von Ethylen (Industrielle Organische Chemie, 2. Auflage, 1978, Verlag Chemie, Seite 217 bis 223).

Als Harnstoffe II seien bevorzugt genannt: Ethylen- und Propylenharnstoff.

Auch diese Verbindungen sind käuflich oder nach bekannten Methoden erhältlich.

Die Verfahrensprodukte der Formel I sind gesuchte Zwischenprodukte, z.B. für vernetzte Polymere (DE-A-35 43 348).

### Beispiele

### Beispiel 1

### N,N'-Divinylpropylenharnstoff

15 g (0,15 mol) Propylenharnstoff und 12 g (0,1 mol) 4-N,N-Dimethylaminopyridin wurden vorgelegt, auf 95°C erhitzt, innerhalb von 15 Minuten 25,8 g (0,26 mol) Vinylpropionat zugetropft und 5,5 Stunden unter Rückfluß erhitzt.

Nach destillativer Aufarbeitung erhielt man 20,3 g (89%) N,N'-Divinylpropylenharnstoff; Sdp.: .... bis ...°C.

### Beispiel 2

### N,N'-Divinylethylenharnstoff

43 g (0,5 mol) Ethylenharnstoff und 30,5 g (0,25 mol) 4-N,N-Dimethylaminopyridin wurden in 100 g Toluol vorgelegt, auf 100°C erhitzt, innerhalb von 30 Minuten 110 g (1,1 mol) Vinylpropionat zugetropft und 5 Stunden unter Rückfluß erhitzt.

Nach destillativer Aufarbeitung erhielt man 63,6 g (91 %) N,N'-Divinylethylenharnstoff; Sdp.:

## Patentansprüche

1. Verfahren zur Herstellung von N-Alkenylharnstoffen der allgemeinen Formel I 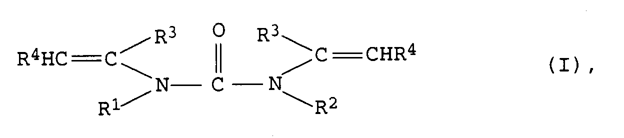 in der
R¹,R² Wasserstoff, C₁- bis C₄₀-Alkyl, C₂- bis C₄₀-Alkenyl, C₃- bis C₂₀-Cycloalkyl, C₄- bis C₂₀-Alkyl-cycloalkyl, C₄- bis C₂₀-Cycloalkyl-alkyl, Aryl, C₇- bis C₂₀-Alkylaryl, C₇- bis C₂₀-Aralkyl, durch C₁- bis C₈-Alkyl, C₁- bis C₈-Alkoxy, Halogen ein- bis fünffach substituiertes Aryl, C₇- bis C₂₀-Alkylaryl, C₇- bis C₂₀-Aralkyl oder gemeinsam eine gegebenenfalls durch C₁- bis C₈-Alkyl ein- bis sechsfach substituierte C₂- bis C₁₀-Alkylendikette und
R³,R⁴ Wasserstoff oder C₁- bis C₈-Alkyl
bedeuten, durch Umsetzung von Harnstoffen der allgemeinen Formel II 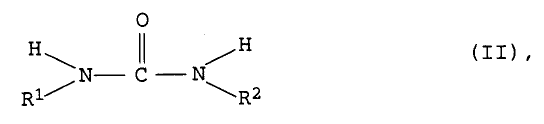 in der R¹ und R² die oben genannten Bedeutungen haben, mit einem Carbonsäurealkenylester der allgemeinen Formel III 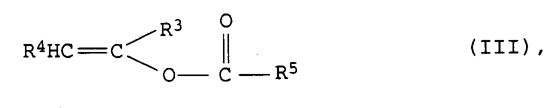 in der R³ und R⁴ die oben genannten Bedeutungen haben und R⁵ Wasserstoff, C₁- bis C₄₀-Alkyl, C₃- bis C₂₀-Cycloalkyl, C₄- bis C₂₀-Alkyl-cycloalkyl, Aryl, C₇- bis C₂₀-Alkylaryl, C₇- bis C₂₀-Aralkyl, durch C₁- bis C₈-Alkyl ein- bis dreifach substituiertes Aryl oder C₇- bis C₂₀-Aralkyl bedeutet, bei Temperaturen von 0 bis 180°C und Drücken von 0,01 bis 10 bar, dadurch gekennzeichnet, daß man die Umsetzung in Gegenwart einer Base durchführt.

2. Verfahren zur Herstellung von N-Alkenylharnstoffen nach Anspruch 1, dadurch gekennzeichnet, daß R³ für Wasserstoff steht.

3. Verfahren zur Herstellung von N-Alkenylharnstoffen nach Anspruch 1, dadurch gekennzeichnet, daß R³ und R⁴ für Wasserstoff stehen.

4. Verfahren zur Herstellung von N-Alkenylharnstoffen nach den Ansprüchen 1 bis 3, dadurch gekennzeichnet, daß R¹ und R² für Wasserstoff, C₁- bis C₂₀-Alkyl oder gemeinsam für eine gegebenenfalls durch C₁- bis C₄-Alkyl ein- bis dreifach substituiertes C₂- bis C₇-Alkylendikette stehen.

5. Verfahren zur Herstellung von N-Alkenylharnstoffen nach den Ansprüchen 1 bis 4, dadurch gekennzeichnet, daß R¹ und R² für C₁- bis C₁₂-Alkyl oder gemeinsam für eine C₂- bis C₅-Alkylendikette stehen.

6. Verfahren zur Herstellung von N-Alkenylharnstoffen nach den Ansprüchen 1 bis 5, dadurch gekennzeichnet, daß man die Umsetzung bei Temperaturen von 40 bis 150°C durchführt.

7. Verfahren zur Herstellung von N-Alkenylharnstoffen nach den Ansprüchen 1 bis 6, dadurch gekennzeichnet, daß man die Umsetzung bei Temperaturen von 50 bis 120°C durchführt.

8. Verfahren zur Herstellung von N-Alkenylharnstoffen nach den Ansprüchen 1 bis 7, dadurch gekennzeichnet, daß man die Umsetzung bei Drücken von 0,1 bis 2 bar durchführt.

9. Verfahren zur Herstellung von N-Alkenylharnstoffen nach den Ansprüchen 1 bis 8, dadurch gekennzeichnet, daß man die Umsetzung bei Atmosphärendruck durchführt.
